# EUROPEAN PATENT APPLICATION

(11) **EP 4 797 272 A1**
(43) Date of publication of application: **26.08.2026**
(21) Application number: 26155541.1
(22) Date of filing: 30.01.2026
(51) Int. Cl.: G16H 15/00, G16H 30/20, G16H 30/40, G16H 50/20, G16H 50/70

(54) **TARGET RADIATION DOSE ESTIMATION FOR MEDICAL IMAGING EXAMS LEVERAGING ARTIFICIAL INTELLIGENCE**

(30) Priority: 24.02.2025 US 202519061251
(71) Applicant: GE Precision Healthcare LLC, Waukesha, WI 53188 (US)
(72) Inventor: BANGALORE SHIVANNACHAR, Rashmi, 560066 Bangalore (IN); PUJADAS, Pilar, 28023 Madrid (ES); NOLAN, Jason, Lauderdale, 33322 (US)
(74) Representative: Kilburn & Strode LLP

(57) **Abstract**

Techniques for estimating target radiation doses for medical imaging exams leveraging artificial intelligence (AI) are described. In an example, a method can comprise training an AI model to estimate target dose information for past medical imaging exams using historical exam reports for the past imaging exams and actual radiation dose information included in the historical exam reports indicating actual radiation dosages exposed to past patients as a result of the past imaging exams. Once trained, the AI model is applied to estimate the target dose information for new medical imaging exams prior to performance of the new exams. The target dose information is further provided to the corresponding imaging systems scheduled for the new exams. In some implementations, the target dose information can control the acquisition protocol used for the exams to ensure compliance with the target dose information.

## Description

### TECHNICAL FIELD

This disclosure relates generally to medical imaging, and more particularly to target radiation dose estimation for medical imaging exams leveraging artificial intelligence (Al).

### BACKGROUND

Many medical imaging modalities use ionizing radiation to generate medical images, including X-ray, computed tomography (CT), fluoroscopy, mammography, and nuclear medicine. Ionizing radiation in medical imaging can be harmful to patients because it has enough energy to remove electrons from atoms, potentially damaging cells and deoxyribonucleic acid (DNA). Excessive or repeated exposure to ionizing radiation from medical imaging exams can increase health risks, including increased risks of cancer, tissue and organ damage, birth defects, and others.

Dose optimization in medical imaging refers to the process of minimizing radiation exposure to patients while maintaining the image quality necessary for accurate diagnosis. The goal is to achieve the lowest possible radiation dose without compromising diagnostic value, following the ALARA principle (As Low As Reasonably Achievable).

Determining the optimal dose for a medical imaging exam is difficult due to multiple factors, including patient differences, image quality requirements, and technological limitations. Generally, for all imaging modalities involving ionizing radiation, there is a trade-off between image quality and radiation dose. Reducing radiation dose increases image noise, which can degrade image quality. However, the acceptable level of image quality depends on the specific clinical task. For example, detecting small lung nodules requires higher image clarity than evaluating a simple bone fracture. In addition, the amount of radiation required to generate images of acceptable diagnostic quality for a specific clinical task varies depending on patient specific factors, including patient size and body composition, age, gender, comorbidities, and others. Further, each imaging modality (X-ray, CT, fluoroscopy, etc.) has different dose optimization challenges. Even within the same modality, different protocols exist for different clinical indications (e.g., a high-resolution chest CT vs. a routine follow-up scan). Furthermore, imaging equipment differs in sensitivity, detector efficiency, and dose modulation capabilities. In addition, radiologists and technologists may have different preferences for image clarity, leading to variations in dose selection. Less experienced operators may err on the side of caution, using higher doses than necessary to ensure image quality.

### SUMMARY

The following presents a simplified summary of the specification in order to provide a basic understanding of some aspects of the specification. This summary is not an extensive overview of the specification. It is intended to neither identify key or critical elements of the specification, nor delineate any scope of the particular implementations of the specification or any scope of the claims. Its sole purpose is to present some concepts of the specification in a simplified form as a prelude to the more detailed description that is presented later.

According to an embodiment, a system includes at least one memory that stores computer-executable components, and at least one processor that executes the computer-executable components stored in the at least one memory. The computer-executable components can comprise a communication component that receives new exam information for a new medical imaging exam to be performed on a patient, and a matching component that accesses a tracking database comprising historical exam reports for past medical imaging exams performed on past patients and identifies one or more target medical imaging exams of amongst the past medical imaging exams that correspond to a match to the new medical imaging exam based on defined similarity criteria between the new exam information and the historical exam reports. The computer-executable components further comprise a target dose estimation component that applies input parameters extracted from the new exam information and one or more historical exam reports for the one or more target medical imaging exams as input to a dose estimation model, and generates target dose information for the new medical imaging exam information as output, wherein the target dose information indicates one or more recommended radiation dose measures for the new medical imaging exam, and wherein the dose estimation model comprises an artificial intelligence (AI) model trained on the historical imaging exam reports.

In one or more implementations, the computer-executable components further comprise a recommendation component that provides the target dose information to an imaging system scheduled for the new imaging exam prior to performance of the new medical imaging exam on the patient. In some implementations, the computer-executable components further comprise a configuration component that determines configuration information for the imaging system that facilitates achieving the one or more recommended radiation dose measures, and controls usage of the configuration information by the imaging system for the performance of the new medical imaging exam on the patient.

In one or more embodiments, the computer-executable components further comprise a training component that trains the dose estimation model using the historical exam reports and actual radiation dose information included in the historical exam reports indicating actual radiation dosages exposed to the past patients as a result of the past imaging exams.

In some embodiments, elements described in connection with the disclosed systems can be embodied in different forms such as a computer-implemented method, a computer program product, or another form. For example, in another embodiment, a computer-implemented method can comprise receiving, by a system comprising a processor, new exam information for a new medical imaging exam to be performed on a patient, accessing, by the system, a tracking database comprising historical exam reports for past medical imaging exams performed on past patients, and identifying, by the system, one or more target medical imaging exams of amongst the past medical imaging exams that correspond to a match to the new medical imaging exam based on defined similarity criteria between the new exam information and the historical exam reports. The method can further comprise applying, by the system, input parameters extracted from the new exam information and one or more historical exam reports for the one or more target medical imaging exams as input to a dose estimation model, and generating, by the system in response to the applying, target dose information for the new medical imaging exam information, wherein the target dose information indicates one or more recommended radiation dose measures for the new medical imaging exam, and wherein the dose estimation model comprises an artificial intelligence model trained on the historical imaging exam reports.

In another embodiment, a non-transitory machine-readable storage medium can comprise executable instructions that, when executed by a processor, facilitate performance of operations, comprising: receiving new exam information for a new medical imaging exam to be performed on a patient; accessing a tracking database comprising historical exam reports for past medical imaging exams performed on past patients; identifying one or more target medical imaging exams of amongst the past medical imaging exams that correspond to a match to the new medical imaging exam based on defined similarity criteria between the new exam information and the historical exam reports; applying input parameters extracted from the new exam information and one or more historical exam reports for the one or more target medical imaging exams as input to a dose estimation model; and generating, in response to the applying, target dose information for the new medical imaging exam information, wherein the target dose information indicates one or more recommended radiation dose measures for the new medical imaging exam, and wherein the dose estimation model comprises an artificial intelligence model trained on the historical imaging exam reports.

### BRIEF DESCRIPTION OF THE DRAWINGS

Numerous aspects, implementations, objects and advantages of the present invention will be apparent upon consideration of the following detailed description, taken in conjunction with the accompanying drawings, in which like reference characters refer to like parts throughout, and in which:
FIG. 1 illustrates a high-level block diagram of an example system that facilitates target radiation dose estimation for medical imaging exams leveraging artificial intelligence (AI), in accordance with one or more embodiments described herein;
FIG. 2 illustrates an example process that facilitates controlling performance of medical imaging exams in accordance with optimal radiation dosages determined for the respective exams, in accordance with one or more embodiments described herein;
FIGs. 3A and 3B present a table describing example input parameters evaluated by a dose estimation model to predict target doses for medical imaging exams, in accordance with one or more embodiments described herein;
FIG. 4 illustrates an example dose management system, in accordance with one or more embodiments described herein;
FIG. 5 illustrates an example process that facilitates improving the performance of a dose estimation model over time, in accordance with one or more embodiments described herein;
FIG. 6 illustrates an example computer-implemented method that facilitates target radiation dose estimation for medical imaging exams leveraging AI, in accordance with one or more embodiments described herein;
FIG. 7 illustrates another example computer-implemented method that facilitates target radiation dose estimation for medical imaging exams leveraging AI, in accordance with one or more embodiments described herein;
FIG. 8 illustrates another example computer-implemented method that facilitates target radiation dose estimation for medical imaging exams leveraging AI, in accordance with one or more embodiments described herein;
FIG. 9 is a schematic block diagram illustrating a suitable operating environment; and
FIG. 10 is a schematic block diagram of a sample-computing environment.

### DETAILED DESCRIPTION

The following detailed description is merely illustrative and is not intended to limit embodiments and/or application or uses of embodiments. Furthermore, there is no intention to be bound by any expressed or implied information presented in the preceding Background section, Summary section or in the Detailed Description section.

As described in the Background Section, dose optimization in medical imaging is challenging due to several factors, including the need to balance image quality with radiation exposure, patient variability, technological limitations, and workflow constraints. With this context in mind, the disclosed subject matter is directed to systems, computer-implemented methods, apparatus and/or computer program products that facilitate estimating target radiation doses for medical imaging exams leveraging artificial intelligence (AI). In an example, a method can comprise training an AI model (referred to herein as a "dose estimation model) to estimate target dose information for past medical imaging exams using historical exam reports for the past imaging exams and actual radiation dose information included in the historical exam reports indicating actual radiation dosages exposed to past patients as a result of the past imaging exams. Once trained, the dose estimation model is applied to estimate the target dose information for new medical imaging exams prior to performance of the new exams. The target dose information is further provided to the corresponding imaging systems scheduled for the new exams. In some implementations, the target dose information can control the acquisition protocol used for the exams to ensure compliance with the target dose information.

In various embodiments, the trained dose estimation model is configured to intelligently fill in gaps and/or adjust input parameters based on the historical data and predefined clinical rules. For instance, if there's a slight difference in the study protocol between the historical database and the current input parameters, the dose estimation model maps the relevant missing factors from the available records without compromising the decision-making process. This case-by-case approach ensures the model remains robust and reliable, even when faced with variations.

In this regard, this ability to automatically estimate the target dose measure for a new exam prior to performance thereof as tailored based on input criteria specific to the new patients (e.g., age, gender, size, body composition, pregnancy status, and other IMDs) and input criteria specific to the new exams (e.g., clinical indication for the exam, area of the body being imaged, imaging system scheduled for the exam, type of the exam, and others) provides significant technical benefits in medical imaging that have yet to be achieved in the past. Although various standards exist that provide diagnostic reference levels (DRLs) with radiation doses used for common imaging exams, these standards merely provide general guidelines and are used to guide radiologists and imaging technicians in association with manually tailoring the radiation dose exposed to patients for actual exams. In this regard, these standards are not tailored based on specific characteristics unique to each imaging exam and patient. On the contrary, the disclosed techniques use AI to learn the optimal radiation dose ranges in terms of effective dose, organ dose and more granular dose metrics tailored to respective imaging modalities, relevant patient factors (e.g., age, gender, size, body composition, past exposure levels, and more), and a variety of different clinical indications, exam types (accounting for different body parts imaged), acquisition protocols, acquisition parameters and various other factors, wherein learning is based on a plethora of different past exams and actual radiation doses exposed to the past patients, enabling the ability accurately estimate tailored target radiation doses for new exams and patients.

In this regard, the ability to automatically estimate highly specific target radiation doses for different types of medical imaging exams prior to performance of these exams can significantly benefit the patients, radiologists and/or imaging technicians and imaging system providers. For example, in various embodiments, the disclosed system can tailor and/or control the acquisition protocol used for the respective exams to ensure the target dose is achieved, thereby minimizing over exposure to patients. In addition, the target radiation dose information can be used to regulate adjusting acquisition parameters in real-time during the exam by providing alerts when adjustments result in exposure beyond the target dose or targe dose range. Further, by providing the target dose information to the imaging system scheduled for the exam pre-scan, the imaging workflow is significantly streamlined, saving the radiologist or imaging technician significant time otherwise required to manually estimate the target dose for the patient and the exam. Furthermore, by providing the target dose information pre-scan for an entire network of different imaging systems associated with the same organization as well as disparate organization, the disclosed techniques ensure systematic compliance to standardized dose exposure settings across all imaging systems, regardless of different modalities, makes, models, technician expertise and the like.

The terms "algorithm" and "model" are used herein interchangeably unless context warrants particular distinction amongst the terms. The terms "artificial intelligence (AI) model" and "machine learning (ML) model" are used herein interchangeably unless context warrants particular distinction amongst the terms. Reference to an AI or ML model herein can include any type of AI or ML model, including (but not limited to): deep learning (DL) models, neural network models, deep neural network models (DNNs), convolutional neural network models (CNNs), generative adversarial neural network models (GANs), transformer models, large language models (LLMs) and the like. An AI or ML model can include supervised learning models, unsupervised learning models, semi-supervised learning models, combinations thereof, and models employing other types of ML learning techniques. An AI or ML model can include a single model or a group of two or more models (e.g., an ensemble model, chained models, or the like).

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

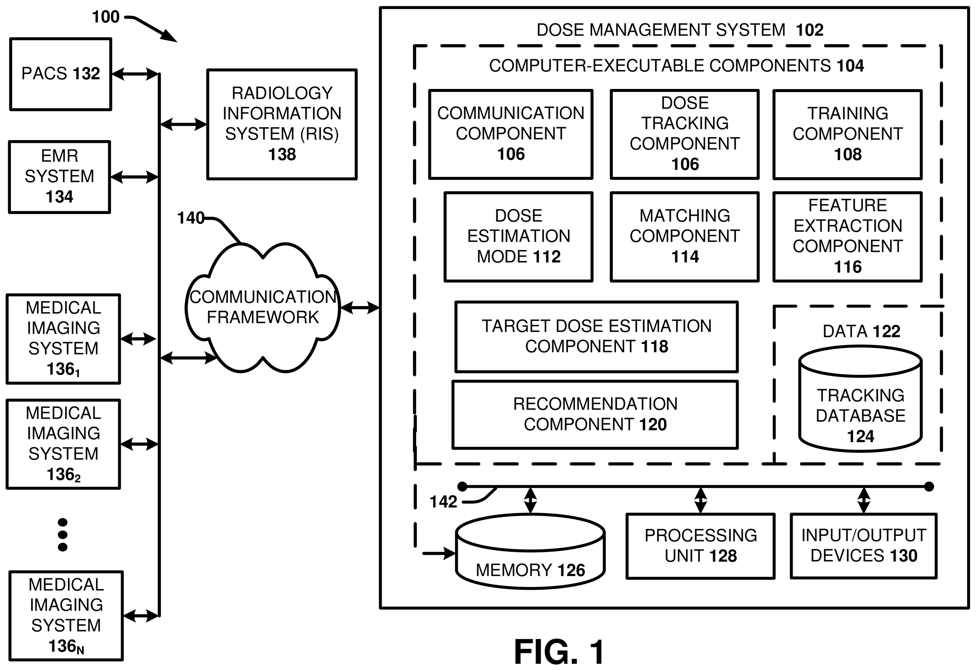

Turning now to the drawings, FIG. 1 illustrates a high-level block diagram of an example system 100 that facilitates target radiation dose estimation for medical imaging exams leveraging AI. System 100 can include or correspond to one or more computing devices, machines, virtual machines, computer-executable components, datastores, and the like that may be communicatively coupled to one another either directly or via one or more wired or wireless communication frameworks. Aspects of the systems, apparatuses or processes explained in this disclosure can constitute computer-executable or machine-executable component(s) embodied within machine(s), e.g., embodied in one or more computer readable mediums (or media) associated with one or more machines. Such component(s), when executed by the one or more machines, e.g., computer(s), computing device(s), virtual machine(s), etc. can cause the machine(s) to perform the operations described.

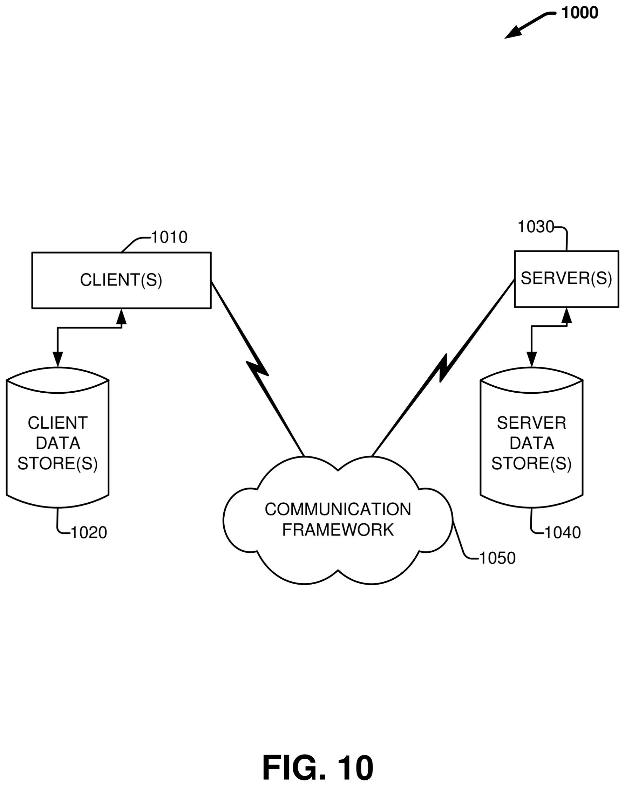

In this regard, system 100 includes dose management system 102, radiology information system (RIS) 138, Picture Archiving and Communication System (PACS 132), electronic medical record (EMR) system (EMR 134), and a plurality of medical imaging systems 136_{1-N}, (the number of which N can vary), respectively connected to one another via communication framework 140. Communication framework 140 can include or correspond to any existing or future developed wired or wireless communication frameworks (examples of which are described with reference to FIG. 10 and communication framework 1050). Dose management system 102, RIS 138, PACS 132, EMR system 134, and medical imaging systems 136_{1-N} can respectively include or correspond to one or more computing devices, machines, virtual machines, computer-executable components, and/or datastores.

In various embodiments, dose management system 102 can include or correspond to a cloud-based medical imaging dose management system that facilitates tracking and controlling the amount of radiation dose exposed to patients via medical imaging exams performed via respective medical imaging systems 136_{1-N}. The medical imaging systems 136_{1-N} can respectively include or correspond to the physical medical imaging devices or machines (i.e., the hardware) used to perform the medical imaging exams as well as the computer hardware and software employed by the machines to control acquisition, image reconstruction, image rendering, image and storage, and the like. In this regard, it should be appreciated that the respective medical imaging systems 136_{1-N} can include or be coupled to one or more computing devices which can further be communicatively coupled to the dose management system 102 and other systems/devices (e.g., PACS 132, EMR system 134, RIS 138, and others) via communication framework 140.

Medical imaging systems 136_{1-N} can include various types of medical imaging system modalities that use ionizing radiation, including X-ray systems, CT systems, fluoroscopy systems, mammography (MG) systems, nuclear medicine systems (e.g., including positron emission tomography (PET) systems and single photon emission computed tomography (SPECT)) systems.), interventional radiology systems, and any other existing or further developed medical imaging system that uses ionizing radiation The medical imaging systems 136_{1-N} can include or correspond to medical imaging systems associated same and disparate entities and organizations (e.g., hospitals, medical enterprises, radiology departments or facilities, institutions or the like) and can include various different types, makes and models of medical imaging systems of the same and disparate imaging modalities. In various embodiments, the medical imaging systems 136_{1-N} can include or correspond to a large number of medical imaging systems distributed across various geographical locations. For example, the medical imaging systems 136_{1-N} can include tens, hundreds, thousands, hundreds of thousands, millions or more of medical imaging systems located at various geographical locations across the world.

In some embodiments, information identifying the respective medical imaging systems 136_{1-N}, the capabilities of the respective medical imaging systems 136_{1-N} (e.g., device type, modality, make/model, acquisition capabilities, post-processing capabilities, etc.) and imaging exams performed and ordered or scheduled for performance at the respective medical imaging systems 136_{1-N} can be provided by RIS 138. A radiology information system (RIS) is a specialized healthcare system used in radiology departments to manage medical imaging records and related data. It plays a crucial role in the workflow of radiology departments, enabling the management of patient data, imaging procedures, results, and administrative tasks.

In some embodiments, RIS 138 corresponds to a centralized RIS that manages medical imaging records and related data associated with all the medical imaging systems 136_{1-N}. For example, in some implementations, RIS 138 can maintain and track information identifying the respective medical imaging systems 136_{1-N}, the make and model (and age) of the respective medical imaging systems, the modality and capabilities of the respective medical imaging systems 136_{1-N}, and imaging exams performed and ordered or scheduled for performance at the respective medical imaging systems 136_{1-N}. In some embodiments, RIS 138 can also include or be integrated with PACS 132, which stores medical image data acquired via respective exams performed at the medical imaging systems136_{1-N}. PACS 132 can also store metadata associated with the medical image data, including information identifying the patients, the acquisition protocol and corresponding acquisition parameters used, the imaging technician that performed the exam, and the like. In this regard, RIS 138 typically handles the administrative and operational aspects of radiology services, while PACS 132 is responsible for storing, retrieving, and viewing medical images. Together, RIS 138 and PACS 132 form the backbone of radiology departments, streamlining both image management and workflow processes. In some embodiments, RIS 138 can also include or be integrated with EMR system 134, which stores electronic medical records for patients, including demographic information (e.g., age, gender, size, height, weight, body composition, body mass index (BMI), etc.), medical history information (e.g., past/current medical conditions, procedures, comorbidities, etc.), medication information, allergy information, and the like.

In accordance with various embodiments, RIS 138 can also control and manage scheduling of medical imaging exams to be performed at respective medical imaging systems 136_{1-N}. In this regard, imaging exam orders are prescribed by an ordering physician after evaluating a patient. The imaging exam order typically includes information identifying the patient (e.g., via a unique identifier (ID) such as the patient's name, or another type of anonymous ID), the ordering physician, the clinical indication for the exam (e.g., a detailed explanation of why the imaging is being ordered, such as the known or suspected diagnosis, symptoms, clinical findings, etc.), the specific modality of the exam (e.g., X-ray, CT, MG, etc.), and the area or region of the body (e.g., referred to as the region of interest, (ROI)) to be imaged (e.g., the specific body part or anatomical region to be imaged).

In accordance with the disclosed techniques, imaging orders for new exams are electronically entered into RIS 138. The exam is then scheduled at a medical imaging system of amongst imaging systems 136_{1-N}, and the scheduling information is provided in the RIS 138 (e.g., date/time of scheduling of the exam, the patient information, and the specific imaging system scheduled for the exam).

Prior to performance of a new exam, the recommended acquisition protocol may be determined for the exam based on the order information (e.g., the modality, the anatomical ROI, the clinical indication, patient parameters, and other factors) and entered into the RIS 138. The imaging exam is then performed using the parameters specified in the protocol. If the technologist or radiologist determines that adjustments are needed (e.g., changing a slice thickness or modifying contrast), they may modify the protocol through RIS 138 or directly on the imaging system. In other embodiments, the technologist/radiologist may manually determine and apply the acquisition protocol used for the exam without receiving a preconfigured or recommended acquisition protocol via the RIS 138 or another system.

The acquisition protocol of a medical imaging exam refers to the standardized set of parameters and procedures used to obtain high-quality images. Each imaging modality (X-ray, CT, MRI, ultrasound, nuclear medicine) has different acquisition protocol capabilities tailored to the specific type of exam (e.g., based on the area of the body being imaged and the clinical indication) and patient characteristics. Acquisition protocols vary significantly between different types of medical imaging exams due to the unique principles, purposes, and technological characteristics of each modality. Acquisition protocols further vary for different types of imaging exams within the same modality because the clinical objectives, anatomy being examined, patient factors, and technical requirements can differ significantly.

Generally, for imaging exams involving ionizing radiation, the acquisition protocol used should be tailored to provide a diagnostically acceptable level of image quality while minimizing radiation dose exposure (for radiation-based modalities such as CT, X-ray, and nuclear medicine). However, determining the optimal dose for a medical imaging exam is difficult due to multiple factors. Generally, for all imaging modalities involving ionizing radiation, there is a trade-off between image quality and radiation dose. Reducing radiation dose increases image noise, which can degrade image quality. However, the acceptable level of image quality depends on the specific clinical task. For example, detecting small lung nodules requires higher image clarity than evaluating a simple bone fracture. In addition, the amount of radiation required to generate images of acceptable diagnostic quality for a specific clinical task varies depending on patient specific factors, including patient size and body composition, age, gender, comorbidities, and others.

Further, each imaging modality (X-ray, CT, fluoroscopy, etc.) has different dose optimization challenges. Even within the same modality, different protocols exist for different clinical indications (e.g., a high-resolution chest CT vs. a routine follow-up scan). Furthermore, imaging equipment differs in sensitivity, detector efficiency, and dose modulation capabilities. In addition, radiologists and technologists may have different preferences for image clarity, leading to variations in dose selection. Less experienced operators may err on the side of caution, using higher doses than necessary to ensure image quality.

With this context in mind, in various embodiments, dose management system 102 can provide for determining the optimal radiation dose or dose range for new medical imaging exams to be performed at respective medical imaging systems 136_{1-N} as tailored based on the unique factors associated with each exam, including (but not limited to), the modality, the clinical indication for the exam, the area of the body being imaged (e.g., the ROI), and patient specific factors including (but not limited to), age, size (e.g., height, weight, BMI, etc.), gender, implants, and others. To facilitate this end, dose management system 102 leverages AI and historical exam reports (e.g., included in tracking database 124) for past medical imaging exams performed on past patients via the respective medical imaging systems 136_{1-N} that provide detailed information regarding the past exams (e.g., imaging system used, modality, acquisition protocols used, type of exam performed, clinical indication for the exam, area of the body imaged, and patient specific factors) as well as dose information that provides one or more measures of the amount of radiation dose exposed to the past patients via the exams.

More particularly, dose management system 102 can train (e.g., via training component 108) one or more AI models (e.g., dose estimation model 112) to estimate target dose information for the past medical imaging exams as a function of relevant input parameters (e.g., exam type, modality, clinical indication for the exam, area of the body imaged, patient specific factors, and various others discussed herein) using the historical exam reports. Once trained, the dose management system 102 can apply the trained version of the dose estimation models 112 to estimate the target dose information for new medical imaging exams received at RIS 138 prior to performance of the new exams. The dose management system 102 can further provide the target dose information to the corresponding imaging systems 136_{1-N} scheduled for the new exams prior to performance of the new exams at the corresponding imaging systems 136_{1-N}. In some implementations, the target dose information can control the acquisition protocol used for the exams to facilitate compliance with the target dose information. Additionally, or alternatively, the target dose information can control the particular medical imaging system (of amongst medical imaging system systems 136_{1- N}) used for the exam. For example, in some implementations, the dose management system 102 can determine and recommend an imaging system of the same modality requested for the exam that may have specific capabilities applicable to achieve the target dose other than that initial scheduled for the exam in implementations in which the scheduled system cannot achieve the target dose under the constraints involved (e.g., patient based constraints and/or image quality constraints).

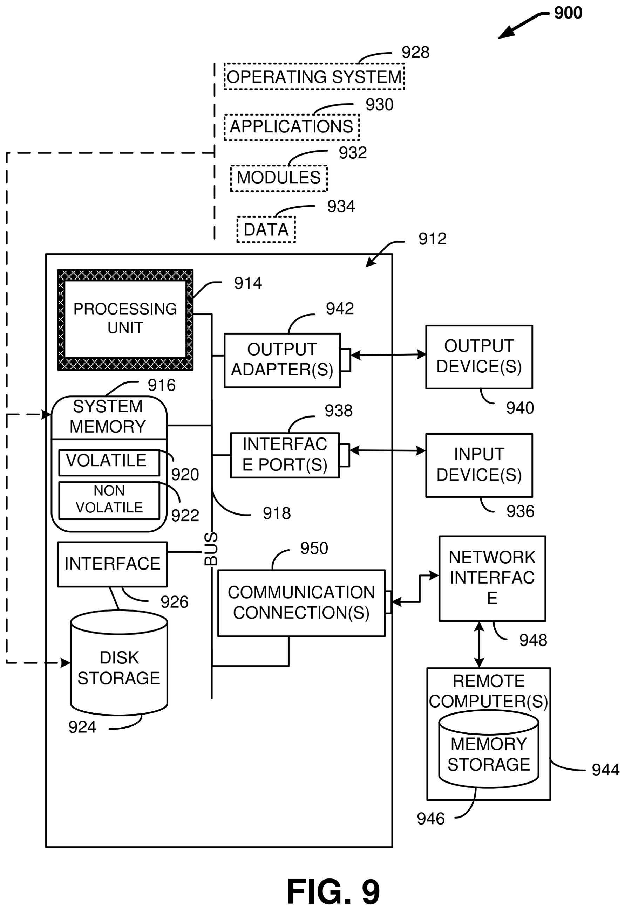

In this regard, dose management system 102 can comprise at least one memory 126 that stores computer-executable components 104, and at least one processor or processing unit 128 that executes the computer-executable components 104 stored in the at least one memory 126. The computer-executable components include, but are not limited to, communication component 106, dose tracking component 108, training component 108, dose estimation model 112, matching component 114, feature extraction component 116, target dose estimation component 118 and recommendation component 120. Examples of said memory 126 and processing unit 128 as well as other suitable computer or computing-based elements, can be found with reference to FIG. 9 (e.g., system memory 916 and processing unit 914 respectively), and can be used in connection with implementing one or more of the components shown and described in connection with FIG. 1, or other figures disclosed herein. Memory 126 can also store data 122 (e.g., information included in tracking database 124) that is received by, used by, and/or generated by the dose management system 102. Additionally, or alternatively, any information that is used by the computer-executable components 104 can be stored at another network accessible device or system (e.g., RIS, PACS 132, EMR system 134, or the like) and accessed by the dose management system 102 via communication framework 140.

Dose management system 102 can further include one or more input/output devices 130 to facilitate receiving user input and rendering data to users in association with performing various operations described with respect to the order computer-executable component 104. Suitable examples of the input/output devices 130 are described with reference to FIG. 9 (e.g., input devices 936 and output devices 940). Dose management system 102 can further include a system bus 142 that couples the memory 126, the processing unit 128 and the input/output devices 130 to one another.

Communication component 106 includes or corresponds to the hardware and/or software that enables wired and/or wireless communication between the dose management system 102 and external systems, devices, machines, databases, etc. (e.g., RIS 138, PACS 132, EMR system 134, medical imaging systems 136_{1-N} and others) via communication framework 140.

In various embodiments, dose tracking component 108 tracks radiation dose information representing one or more measures of actual radiation dosages exposed to patients via performance of medical imaging exams performed via respective medical imaging systems 136_{1-N} and generates corresponding historical exam reports for the performed exams. In this regard, the historical exam reports can provide one or more actual radiation dose measures that represent actual radiation doses exposed to the patients via the exams. These historical exam reports are further stored tracking database 124. More particularly, dose tracking component 108 can acquire or receive dose-related information from the medical imaging systems 136_{1-N} themselves and/or PACS 132 that identifies or indicates one or more measures of actual radiation dosages exposed to patients via medical imaging exams performed via respective medical imaging systems 136_{1-N}. For example, dose tracking component 108 can collect dose-related information from multiple sources, including DICOM^{®} (Digital Imaging and Communications in Medicine) Radiation Dose Structured Reports (RDSR), image headers, DICOM Modality Performed Procedure Steps (MPPS), OCR on dose report images, and other sources.

In some implementations, the actual radiation dose measures can include effective dose, measured in sieverts (Sv), with practical doses often expressed in millisieverts (mSv). The effective dose for an imaging exam involving ionizing radiation is determined by calculating the amount of radiation absorbed by different tissues in the body and weighting it based on the sensitivity of those tissues to radiation-induced effects. Various techniques exist for determining the effective dose for different imaging modalities. For example, the absorbed dose (measured in gray, Gy) quantifies the energy deposited per unit mass of tissue. This can be measured using dose-area product (DAP) for fluoroscopy or CT dose index (CTDI) for CT scan. Different organs and tissues have varying sensitivities to radiation. The International Commission on Radiological Protection (ICRP) assigns tissue weighting factors (W^{t}) to different body parts. For example, more radiation-sensitive tissues (e.g., bone marrow, breast, lung) have higher weighting factors. For practical use, standardized dose conversion coefficients are available from organizations like ICRP and National Council on Radiation Protection and Measurements (NCRP). These factors help estimate effective dose based on dose-length product (DLP) in CT or entrance skin dose in X-ray exam.

The actual radiation dose measures can also include one or more organ doses. Organ dose is the amount of radiation energy absorbed by a specific organ or tissue in the body. It is typically measured in gray (Gy), where 1 Gy = 1 joule per kilogram of tissue. Organ dose varies depending on radiation type, exposure conditions, and tissue composition. Different organs have different sensitivities to radiation; for example, the bone marrow, lungs, and digestive organs are more sensitive than muscle or skin. While organ dose measures the absorbed radiation in a specific organ, effective dose takes into account the biological sensitivity of different organs and the overall risk to health.

The one or more actual radiation dose measures can also include different measures specific to different imaging modalities. For example, as applied to CT, the one or more actual radiation dose measures can include dose length product (DLP), dose index (DI) volume, and size, and specific dose estimate (SSDE). As applied to MG, the one or more actual radiation dose measures can include average glandular dose (AGD). As applied to radio fluoroscopy and interventional radiation, the one or more actual dose measures can include dose areas product (DAP) and Air Kerma Rate (i.e., Kₐᵢᵣr). As applied to interventional procedures, the one or more actual radiation dose measures can include the post exam peak skin dose.

In addition to the one or more actual dose measures, the historical exam reports included in tracking database 124 can include detailed information about the exams performed. This detailed information can include (but is not limited to), the imaging system used (of amongst imaging systems 136_{1-N}), the technician that performed the exam, the modality of the exam, the clinical indication for the exam, the area or region of the body imaged, the specific acquisition protocol and parameters used, resulting imagine quality measures for the exam, the patient and relevant information about the patient (e.g., demographic information, past imaging exams performed, cumulative radiation dose exposed to the patient from the past imaging exams, and other information). In various embodiments, this detailed information included can be provided in accordance with the DICOM standard. In various embodiments, the dose tracking component 106 can extract and/or generate this detailed information from the medical imaging systems 136_{1-N} themselves, the imaging data generated for the medical imaging exams (e.g., as included in PACS 132 and/or RIS 138), and/or corresponding radiology reports (e.g., included in RIS 138), and/or EMR system 134.

For example, in some implementations, the area of the body imaged may be indicated as a function of defined exam types. For example, different types of exams may be defined in accordance with a defined ontology or coding system for respective medical imaging modalities and correspond to different anatomical regions of the body (e.g., Chest CT, head CT, etc.) having predefined DICOM tags. In some implementations, the detailed information can include a local study description (LSD). As used herein, the term "LSD" refers to a specific label, identifier, or metadata field used in medical imaging systems (PACS, RIS, or DICOM headers) to describe a particular imaging study. The LSD can include details like: procedure type (e.g., "Chest X-ray PA", "Abdomen CT with contrast"), patient position (e.g., "Standing", "Supine"), acquisition technique (e.g., "High-resolution", "Low-dose protocol"), and the clinical indication (e.g., "Suspected Pneumonia", "Fracture Evaluation").

In some implementations, the detailed information included in the historical exam reports provided in tracking database 124 can include series description and/or series type data in accordance with the DICOM standard. The series description data can include or correspond to a text label assigned to an imaging series within a study (e.g., found in the DICOM tag (0008,103E) "Series Description"). The series description data provides a brief, human-readable description of the image series, often set by the imaging modality or technologist. The series type data provides a more technical classification that categorizes the imaging series based on modality, technique, and acquisition parameters.

As noted above, the detailed information can also include the specific acquisition protocol and acquisition parameters used for the performed exams. The acquisition protocol is a comprehensive plan for performing an imaging study, while acquisition parameters are the specific technical settings and configurations of the imaging system used during the acquisition process. Acquisition protocols and parameters vary for different modalities and different types of imaging exams within the same modality. The acquisition protocol for instance may include information describing the sequency type (e.g., CT angiography), patient positioning instructions (e.g., supine, prone), and procedure steps (e.g., contrast injection, breath holding instructions, etc.). Acquisition parameters directly impact the image quality, contrast, resolution, scan time and radiation exposure. They are usually fine-tuned by the radiologic technologist based on clinical needs and the patient's condition. Some examples of acquisition parameters include exposure settings (e.g., X-ray tube voltage in CT or mAs in X-ray); slice thickness (e.g., 5mm, 10mm); pitch, scan range (e.g., head, chest, abdomen, scan duration; Field of view (FOV); contrast injection settings (if applicable, such as contrast dosage and timing), image receptor settings, collimation and field size, and others. In some embodiments, the acquisition parameters can include or correspond to the actual console configuration of the imaging system used for an exam.

The information included in the historical exam reports in the tracking database 124 can also include relevant patient factors, including but not limited to, age, height, body composition (e.g., BMI), gender, comorbidities, IMD details and prior imaging study information (e.g., regarding repeat exams of the same type, and prior radiation dose exposure). The patient information can also include information identifying or indicating additional past imaging exams performed on the patient, including cumulative radiation doses exposed to the patient over time based on the past imaging exams. Patient factors play a significant role in determining the acceptable radiation dose for medical imaging procedures that use ionizing radiation, such as X-rays, CT scans, and nuclear medicine. Tailoring the radiation dose to the individual patient is important to ensure diagnostic accuracy while minimizing unnecessary exposure to radiation risks.

For example, children are more sensitive to radiation because their cells are dividing more rapidly, making them more susceptible to radiation-induced DNA damage. Moreover, they have a longer expected lifespan, increasing the time for radiation-induced effects (like cancer) to manifest. Due to these risks, pediatric imaging protocols are adjusted to use the lowest possible radiation dose that still provides sufficient image quality. While older adults are less susceptible to the long-term risks of radiation-induced cancer due to their shorter remaining lifespan, they may have age-related health conditions that necessitate careful dose management. In addition, many elderly patients have comorbidities (e.g., cardiovascular disease, osteoporosis) that require frequent imaging procedures. Cumulative radiation exposure over multiple studies may increase their risk of adverse effects, so dose management remains important. Body size and composition also influence acceptable radiation doses for imaging exams. In larger patients, more radiation is often needed to penetrate the body and produce a clear image. Depending on the exam type, higher doses for obese patients are often necessary to avoid grainy or poor-quality images that could miss critical diagnostic details. However, efforts are made to minimize the dose by optimizing settings such as adaptive dose modulation (which adjusts the dose in real-time based on body part thickness). On the other hand, smaller or leaner patients generally require lower doses because there is less tissue for the radiation to penetrate, and lower photon counts can still produce clear images. Overexposure in these patients can result in unnecessary radiation doses.

Appropriate dose levels for different imaging exam types can also vary based on gender. For example, females are generally more sensitive to radiation-induced cancer, particularly breast and thyroid cancers. As a result, extra precautions may be taken to minimize radiation dose in female patients, especially when imaging these sensitive areas. Pregnancy status of females can also influence the amount of acceptable radiation dose. The developing fetus is highly sensitive to radiation, especially during the first trimester, when organs and tissues are forming. Even small doses of ionizing radiation can increase the risk of developmental abnormalities, miscarriage, or childhood cancer. For pregnant women, alternative imaging modalities that do not use ionizing radiation (such as ultrasound or MRI) are preferred whenever possible. If a radiation-based scan is necessary, protocols should be adjusted to use the lowest possible dose, and lead shielding is used to protect the fetus. In some embodiments involving pregnant women, the actual radiation dose information can also include a measure of radiation dose exposed to the fetus in addition to the mother.

The information included in the historical exam reports can also include presence and concentration information for exams using intravenous (IV) contrast injection. Presence and concentration information in medical imaging exams refers to data that describes the presence and amount (concentration) of contrast agents or other substances in the body during an imaging procedure. This information is crucial for interpreting images, particularly in diagnostic modalities like CT, MRI, and X-ray where contrast agents are often used to enhance visibility of specific tissues, blood vessels, or abnormal areas. Presence information describes whether or not a particular substance, such as a contrast agent or tracer, is detected or present within the body during an imaging study. This information is often a binary indication (yes/no) of whether the substance was injected or ingested, and whether it is in the area of interest. Concentration information refers to the amount of contrast agent or tracer present in the body or in a specific tissue or organ. It is typically expressed in units like milligrams per milliliter (mg/mL), millimoles per liter (mmol/L), or similar units, depending on the imaging modality and the contrast agent used.

The information included in the historical exam reports can also include image quality information that provides one or more measures of resulting quality of the images generated for the performed exams. In some embodiments, quality metrics for the respective historical medical imaging exams can include one or more subjective quality measures, such as an interpretive measure of quality such as a quality score provided by the reviewing physician/radiologist such indicating whether and to what degree the quality of the images was diagnostically acceptable or not. Additionally, or alternatively, the measures of quality can include one or more objective quality measures, such as measure of the amount of noise (e.g., measured as a function of signal-to-noise ratio (SNR) or a similar metric) for respective images of the exam. For example, in some implementations, the quality measure can include a study noise score that represents the amount of background noise within the images at the organ level per image acquired in the study. Still in other implementations, the one or more measures of quality can include a contrast measure (e.g., measured as a function of contrast-to-noise ratio (CNR) or a similar metric), a measure of temporal resolution and/or spatial resolution, and/or a measure regarding the amount, severity and/or type of artifacts (e.g., measured as a function of an amount and/or severity of artifacts such as blurring, streaks, and other types of artifacts) and/or other objective quality measures. In some implementations, the quality information for the performed imaging exams can include or correspond to a quality score or rating that accounts for one or more of these metrics and/or a subjective interpretation of the images as provided by a reviewing entity (e.g., a radiologist or the like) and/or an image quality scoring algorithm.

In some embodiments, the information included in the historical exam reports can also include information regarding radiation dose alerts generated during the imaging exams. For example, real-time dose monitoring technology may be used during performance of the imaging exams that continuously monitors the radiation dose being applied during imaging procedures in real-time. This technology is designed to provide smart alerts when radiation doses are potentially too high for the procedure, helping prevent overexposure.

The information included in the historical exam reports can also include information indicating whether each exam involves a repeat element. As used herein, the term "repeat element" refers to whether the exam or specific images in the exam were repeatedly acquired (usually due to insufficient quality as a result of patient movement, manual error by the technician and/or improper acquisition parameters). Repeat imaging increases radiation exposure, so monitoring the number of repeated scans is important for radiation dose management. The repeat element information captures the frequency and reasons for repetition (if applicable), allowing technologists and radiologists to understand why certain exams and/or image views need to be redone.

The information included in the historical exam reports can also include operator or technician provided information describing a rational or justification for why certain acquisition protocols were applied that are outside recommended acquisition protocols. In particular, many imaging systems include intelligent configuration software that raises warnings or alerts when acquisition protocols tuned by the technician deviate from predefined rules and configuration settings. For example, such warnings or alerts may be based on the selected scan FOV exceeding a threshold level that would result in a longer scan duration and thus a higher radiation dose exposed to the patient. Often times, the technician has a reasonable justification for applying such protocol configurations, such as the patient having a particular unusual anatomy, or particular health condition that warrants usage of the particular protocols. When applicable, this information can also be included in the corresponding historical exam reports.

In various embodiments, training component 108 can employ the historical exam reports included in the tracking database 124 to train and develop a dose estimation model 112 configured to estimate one or more target dose measures for the past exams (or a subset of the past exams used for the model testing and/or validation phases). Once trained the target dose estimation component 118 can apply the trained versions of the dose estimation model 112 to estimate the one or more target dose measures for new exams ordered and scheduled in the RIS 138 prior to performance of the new exams.

In this regard, dose estimation model 112 can include or correspond one or more ML or AI models. The type of the ML or AI models can vary. For example, the dose estimation model 112 can include or correspond to deep learning (DL) models, neural network models, deep neural network models (DNNs), convolutional neural network models (CNNs), generative adversarial neural network models (GANs), transformer models, and the like. In some embodiments, the dose estimation model can include a large language model (LLM). In some embodiments, the dose estimation model 112 can additionally or alternatively include linear regression models, random forest models, gradient boosting models and/or rule based models.

At a high level, the training process involves training the dose estimation model 112 to estimate one or more defined target doses measures (e.g., the model output parameters) for a given exam based on relevant input parameters for the exam and predefined rules (e.g., based on clinical expertise and existing clinical guidelines) integrated within the dose estimation model 112 that defines relationships between the relevant input parameters and predefined constraints on those relationships. To this end, the relevant input parameters and the predefined rules can account for a variety of different imaging exam types with respect to modality, area of the body imaged, clinical indications, patient factors, acquisition protocols, imaging system capabilities, and various other parameters discussed herein. In various embodiments, the target dose measures can include or correspond to the actual dose measures provided for the historical exams included in the tracking database 124. For example, in some embodiments, the one or more target dose measures can include organ dose and/or effective dose. Additionally, or alternatively, the one or more target dose measures can include modality specific target dose measures, such as but not limited to, DLP, CTDI, SSDE, AGD, DAP, Kₐᵢᵣr, and the like. In some embodiments, the one or more target dose measures output by the dose estimation model 112 can include target dose ranges (e.g., including upper and lower bounds on target effective dose, target organ doses, and the like).

In various embodiments, the relevant input parameters can be predefined and include or correspond to the detailed information provided in the historical exam reports for the past exams described above. The relevant input parameters can vary based on exam type (e.g., as a function of modality, area of the body imaged and clinical indication for the exam). For example, in some embodiments, the relevant input parameters for CT exams can include modality, LSD, area of scan/scan type, series description and series type, acquisition protocol, acquisition parameters, defined patient factors (e.g., age, height, BMI, body composition, gender, health status, IMD presence/absence, IMD type, etc.), actual console configuration, one or more defined image quality measures, smart observation information, repeat element information, and dose check justification information.

In some embodiments, the training component 112 can initially review and filter the historical exam reports included in the tracking database 124 to remove exam reports having actual dose measures that exceed acceptable values for the particular type of exam and patient profile (e.g., based on patient age, gender, size, etc.). For example, the acceptable values or value ranges can be based on predefined standards defined for the respective types of imaging exams as defined based on modality, area of scan, clinical indication for the scan, and patient factors (e.g., age, height, BMI, gender, etc.). In some embodiments, the training component 112 can also initially review and filter the historical exam reports included in the tracking database 124 to remove exam reports having quality measures below acceptable values for such quality measures.

The training process employed by the training component 108 can include a supervised machine learning process, an unsupervised machine learning process, or a semi-supervised machine learning process. In this regard, the historical exam reports can represent a wide range of different input criteria variations corresponding to different medical imaging exams performed in the past. For example, the historical exam reports can provide a wide range of different types of medical imaging exams corresponding to different modalities, different imaging systems of amongst the medical imaging systems 136_{1-N}, different LSDs, different scan areas, different patient profiles (e.g., with respect to patient age, height, BMI, gender, age, etc.), different console configurations, different image quality measures, acquisition protocols and parameters, different repeat elements, and so on. In some embodiments, the training component 108 can train a single dose estimation model 112 configured to account for any type of medical imaging exam involving ionizing radiation regardless of modality, exam type, area of scan, acquisition protocol and parameters, patient characteristics and other input criteria. In other embodiments, the training component 108 can train separate dose estimation models corresponding to dose estimation model 112 yet tailored to different groupings of the past exams, as grouped based on one or more of the input parameters (e.g., modality, exam type, area of scan, etc.). With these embodiments, once trained, the target dose estimation component 118 can select and apply the corresponding dose estimation model applicable to each new imaging exam based on the corresponding grouping factor or factors for which the respective models are tailored.

In either of these embodiments, the training process generally involves training the dose estimation model 112 to learn the target dose measures for these past exams based on the multitude of variations across these input parameters, using the actual (and acceptable) dose measure values or value ranges provided for the past exams as the reference or ground truth information. During training, the training component 108 defines and adjusts the dose estimation model parameters (e.g., model weights and biases) based on learned relationships between the input parameters and the acceptable radiation dose values for the respective past exams using a suitable loss function. Once trained on a plethora of different input parameter combinations provided by the historical exam reports included in the tracking database 124, the learned relationships become embedded within the model itself. In this regard, once trained the dose estimation model 112 can be applied to estimate target dose information for new exams with new combinations of input parameters, including new combinations that may not have been represented in the training data.

In some embodiments in which the target dose estimation model 112 includes or corresponds to an LLM, the target dose estimation model can be trained to determine the optimal radiation dose measure(s) for a given patient and clinical use case by leveraging historical the historical exam reports included in the tracking database which provide actual radiation dose measure(s) for past use cases, predefined input parameters and, predefined clinical rules and constraints defined for various types of clinical use cases . In some implementations of these embodiments, the training process integrates supervised learning, reinforcement learning, and retrieval-augmented generation (RAG) to enhance dose optimization.

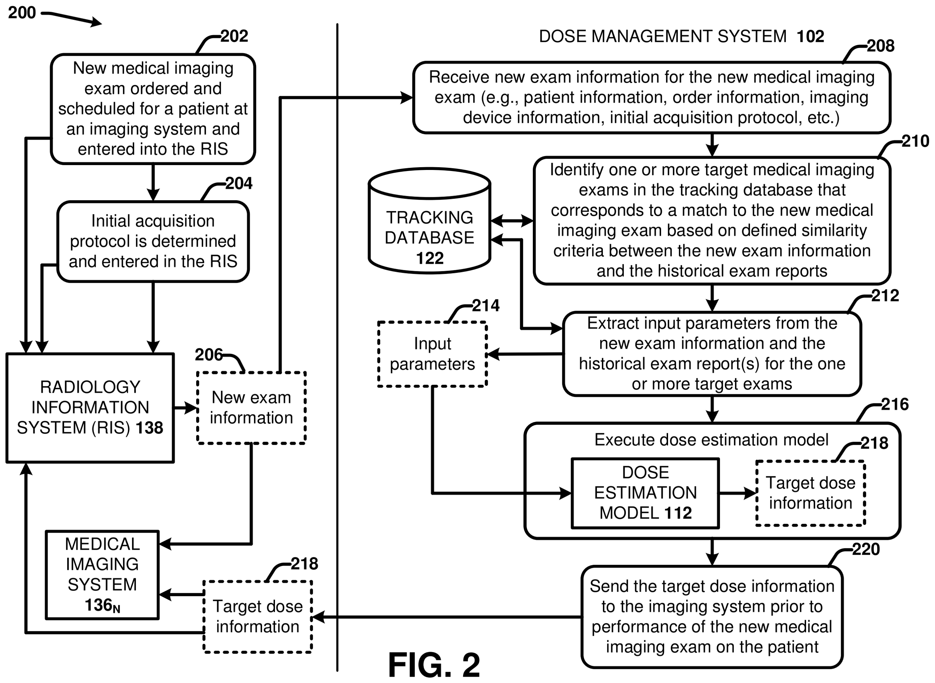

In this regard, in various embodiments, the target dose estimation component 118 can apply the trained version of the dose estimation model 112 to input parameters for new medical imaging exams received at the RIS 138 to generate one or more target dose measures for the new exams prior to performance of the new exams. To facilitate this end, the target dose estimation component 118 can employ matching component 114, feature extraction component 116 and the historical exam reports included in the tracking database 124. The features and functionalities of these components as applied to estimate target dose information for new exams are described with reference to FIG. 2 and process 200.

FIG. 2 illustrates an example process 200 that facilitates controlling performance of medical imaging exams in accordance with optimal radiation dosages determined for the respective exams, in accordance with one or more embodiments described herein. With reference to FIG. 2 in view of FIG. 1, process 200 demonstrates a holistic workflow for processing a new medical imaging exam via system 100. In accordance with process 200, at 202 a new medical imaging exam is ordered and scheduled for a patient at a medical imaging system (e.g., of amongst the medical imaging systems 136_{1-N}) and entered into the RIS 138. The new medical imaging exam can include or correspond to any type of medical imaging exam modality involving ionizing radiation (e.g., X-ray, CT, MG, nuclear medicine, fluoroscopy, interventional radiology, etc.). At this point, the information entered into the RIS 138 for the new exam may include or correspond to information included in the exam order provided by the prescribing physician, such as the exam type, the modality, the area of the body or ROI for the exam, and the clinical indication for the exam. The information entered into the RIS 138 will also include information identifying the patient (e.g., via a unique patient ID) and information identifying the particular medical imaging system (e.g., via an imaging system ID or the like) scheduled for the exam. For ease of description, this particular imaging system is arbitrarily identified in FIG. 2 as medical imaging system 136 _{N}. In some embodiments, relevant patient factors for the patient (e.g., age, size, body composition, gender, pregnancy status, IMDs, etc.) may be extracted from the patient medical record (e.g., in EMR system 134) and entered into the RIS along with the order information at this time.

At 204, the initial acquisition protocol for the exam may be determined and entered into the RIS. For example, in some embodiments, the initial acquisition protocol may be manually determined and entered by a radiologist, a trained technician, or the like, based on the order information. In other embodiments, the initial acquisition protocol may be automatically generated/selected based on the order information. In this regard, the initial acquisition protocol for an imaging exam may correspond to a predefined, standard acquisition protocol determined for the modality, the ROI, the clinical indication and possibly patient factors. For example, many clinical guidelines and/or medical imaging system providers provide preconfigured standardized protocols tailored to specific imaging systems and clinical scenarios. Additionally, or alternatively, the initial acquisition protocol may correspond to a manually defined protocol (e.g., provided by a radiologist) and/or an automatically generated protocol generated via intelligent protocol planning optimization software. For example, in many clinical scenarios, a radiologist reviews the order information and determines the recommended protocol based on the modality, the clinical question, the ROI, patient-specific factors (e.g., age, size, body composition, gender, IMDs, pregnancy status, etc.) and institutional guidelines and best practices. In some implementations, the radiologist may tailor a standardized protocol defined for a particular modality, ROI, clinical indication and in some implementation's patient factors (e.g., age, gender, patient size, etc.) for a particular patient and order, or leave the standardized protocol unmodified.

In other implementations, intelligent protocol management software can automatically determine the recommended or planned protocol based on the order information. Software that automatically generates the optimal acquisition protocol for a medical imaging exam typically leverages advanced algorithms, clinical guidelines, patient data, and imaging system capabilities. These tools aim to standardize and optimize imaging protocols to ensure diagnostic accuracy, efficiency, and patient safety, often leveraging AI. For example, intelligent protocol management software is provided by various medical imaging device providers that automate protocol selection based on patient characteristics and clinical indications. These software systems use AI and machine learning to guide technologists in selecting optimal imaging parameters based on the clinical context, employ preloaded and customizable protocols, and provide for automatic parameter adjustments based on patient size and clinical indication.

In this regard, in various embodiments, once a medical imaging exam is scheduled in the RIS 138, a healthcare provider (radiologist, technologist, or physician) selects or customizes an acquisition protocol for the exam and/or the initial acquisition protocol is automatically defined using AI technology. The initial protocol defines specific parameters for the imaging exam, such as slice thickness, contrast use, resolution, and scanning techniques. The initial acquisition protocol is entered into RIS 138 along with other relevant patient and exam information.

Still in other embodiments, the initial acquisition protocol and/or acquisition parameters to be used for the exam may not be included in the new exam information 206.

In this regard, new exam information 206 corresponds to any information available for the new exam as entered into RIS 138 prior to the performance of the exam, and can include but is not limited to, the order information, the patient information (including relevant patient demographic factors), the information identifying the medical imaging system 136 _{N} at which the exam is scheduled, and the initial acquisition protocol including one or more acquisition parameters.

In some embodiments, the new exam information 206 can be sent directly to the medical imaging system 136 _{N} scheduled for the exam via communication framework 140 (typically using DICOM standards). Additionally, or alternatively, the dose management system 102 can receive, collect or otherwise extract the new exam information 206 once entered into the RIS 138 and prior to performance of the exam.

At 208, the dose management system 202 receives the new exam 206 information for the new medical imaging exam (e.g., via communication component 106). In various embodiments, the parameters included in the new exam information 206 only include or correspond to a subset of the input parameters evaluated by the dose estimation model 112. With these embodiments, the dose management system 102 uses the historical exam reports included in the tracking database 122 to estimate the missing input parameters (e.g., excluded from the new exam information 206 and otherwise not provided in the EMR system 134, the PACS 132 and/or the RIS 138).

More particularly, at 210, the matching component 114 identifies one or more target medical imaging exams in the tracking database 122 that corresponds to a match to the new medical imaging exam based on defined similarity criteria between the new exam information 206 and the historical exam reports. For example, in some implementations, the matching component 114 can identify a single target historical exam report for a past exam that has the closest similarity between the subset of input parameters included in the new exam information and the corresponding input parameters in the target historical exam report.

For example, let's assume the new exam information includes: 1. Patient information for the new patient, including the patient's name and/or a unique anonymous ID for the patient, the patient's age or birthdate gender, height, weight, size, BMI, pregnancy status and IMD information (e.g., regarding presence or absence of IMDs, location and type); 2. Order information identifying the exam type, modality, clinical indication for the exam, and ROI for the exam, and 3. The initial acquisition protocol. In accordance with this example, each component of the new exam information (e.g., components 1-3) includes several sub-components. In accordance with this example, the matching component 114 can find the closest match to the new exam information based on an aggregate measure of similarity between each component and sub-component of the new exam information 206 and the corresponding components and sub-components for the past exams.

In some embodiments, the matching component 114 can generate similarity scores representing measures of similarity between the new medical imaging exam and past imaging exams of the same modality based on aggregate measures of similarity between the new exam information and the corresponding exam information for the past exams. In some implementations of these embodiments, the similarity scoring function employed by the matching component 114 can provide a tailored weighting scheme for defined parameters included in the new exam information. For example, in some implementations, the weighting scheme can weight similarity between patient parameters higher than similarity between initial acquisition protocol parameters. In some embodiments, the matching component 114 can select a single target exam with the highest similarity score. In other embodiments, the matching component 114 can select two or more historical exam reports based on their similarity scores exceeding a threshold similarity score.

At 212, the feature extraction component 116 extracts input parameters 214 from the new exam information 206 and the historical exam report(s) for the one or more target exams. These input parameters 214 correspond to the defined set of input parameters evaluated by the dose estimation model 112. In this regard, as noted above, in some embodiments, the new exam information 206 only provides a subset of the input parameters 214. To this end, at 212, the feature extraction component 116 extracts the remaining subset of input parameters required by the dose estimation model 112 from the one or more historical exam reports for the one or more target exams. In implementations in which two or more target historical exam reports are selected, the feature extraction component 116 can select some of the missing input parameters from each of the two or more exams randomly. Additionally, or alternatively, the feature extraction component 116 can employ a predefined feature selection protocol that compares different values for same parameters included in the respective historical exams and selects one of the values that satisfies predefined selection criteria.

In some implementations, the feature extraction component 116 can select one or more acquisition parameters to be used for the new exam from the one or more similar historical exam reports. For example, in some implementations in which the new exam information 206 excludes one or more acquisition parameters, the feature extraction component 116 can intelligently identify and extract the corresponding acquisition parameters from the one or more similar historical exam reports that facilitate minimizing radiation dose exposed to the patient in accordance with predefined and/or learned selection criteria. Additionally, or alternatively, in some implementations in which the new exam information 206 includes one or more acquisition parameters, the feature extraction component 116 can adjust or replace these parameters with alternative acquisition parameters extracted from the one or more similar historical exam reports based on the predefined and/or learned selection criteria.

In some embodiments in which the dose estimation model 112 includes or corresponds to an LLM, the functionality of the matching component 114 and the feature extraction 116 can collectively include or correspond a RAG process. RAG is an AI framework that enhances estimations performed by an LLM by integrating a retrieval mechanism. Instead of relying solely on a LLM's pre-trained knowledge, RAG dynamically retrieves relevant information from external sources (such as the historical exam reports included in the tracking database 122) and incorporates it into the generated response. With these embodiments, the matching component 114 identifies one or more historical exam reports based on defined similarity criteria between the historical exam reports and the new exam information 206. The similarity criteria can be based on one or more input features included in the new exam information 206. The feature selection component 116 then selects missing input parameters (or parameter values) required by the dose estimation model from the one or more historical exams based on defined rules and/or selection criteria, such as selecting a particular acquisition protocol and/or one more acquisition parameters of amongst the similar exams that is attributed to lower radiation dose exposure relative to other acquisition protocols in the one or more similar exams. In some implementations, even if certain input features are included in the new exam information 206, the feature selection component 116 can be configured to replace one or more of the given input features with a corresponding historical feature value extracted from the one or more similar exams based on defined selection criteria, such as replacing an initial acquisition protocol and/or one or more acquisition parameters for the exam included in the new exam information 206 with a more preferred acquisition protocol of amongst the similar exams that better achieves minimizing radiation dose exposure, in accordance with predefined and/or learned selection criteria. With these implementations, in addition to outputting the target dose information 218, the dose estimation model 112 can also generate output information identifying modified input parameters (e.g., modified acquisition protocols and/or acquisition parameters) selected and recommended for the exam.

As noted above, the collective set of input parameters 214 required for the dose estimation model are predefined and tailed based on the specific exam type. For example, as applied to CT exams the input parameters 214 can include but are not limited to: modality, LSD, area of scan/scan type, series description and series type, acquisition protocol, acquisition parameters, defined patient factors (e.g., age, height, BMI, body composition, gender, health status, IMD presence/absence, IMD type, etc.), actual console configuration, one or more defined image quality measures, smart observation information, repeat element information, and dose check justification information.

In embodiments, in which the dose estimation model 112 comprises one or a plurality of different instances of dose estimation models tailored to different modalities or other clinical scenarios, the predefined input parameters can vary for the respective dose estimation models. With these embodiments, the target dose estimation component 118 can select the applicable dose estimation model based on the new exam information 206 prior to feature selection at 212, and the feature selection component 116 can exact the input parameters 214 according to the defined set of input parameters tailored for the applicable dose estimation model.

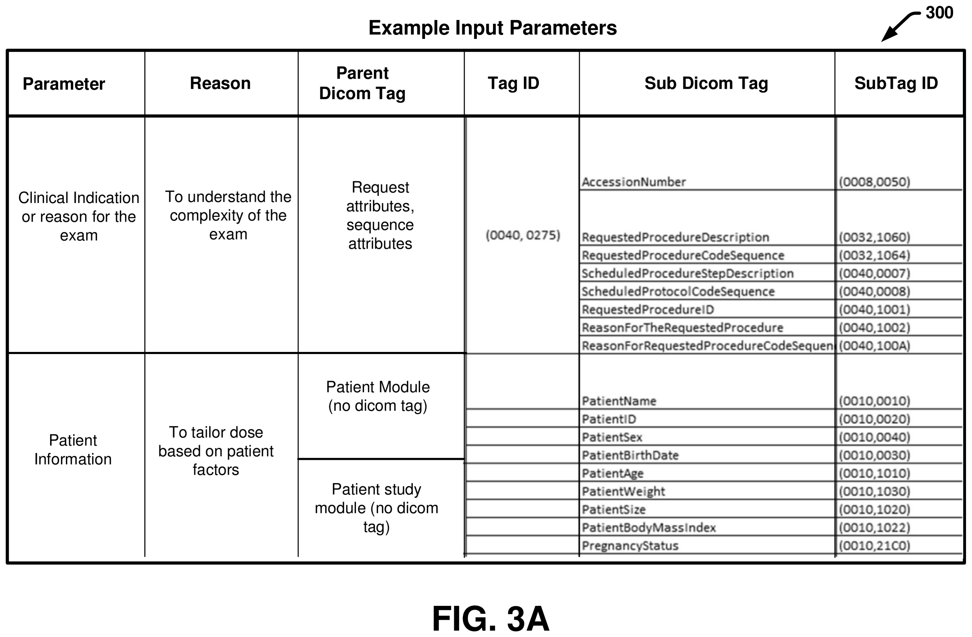

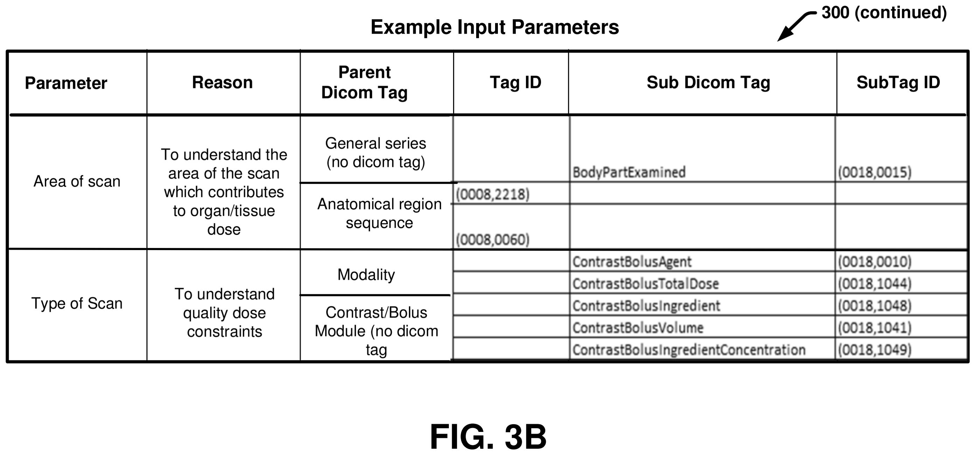

FIGs. 3A and 3B present a table 300 describing some example input parameters evaluated by a dose estimation model 212 to predict target doses for medical imaging exams, in accordance with one or more embodiments described herein. Table 300 provides a general description of four types of input parameter categories, including 1. Clinical indication or reason for the exam, 2. Patient information, 3. Area of the scan and 4. Type of scan. As noted above, the input parameters for the dose estimation model 112 can include many more parameters additional to these input parameter categories and within each input parameter category. The second to the last column of table 300 includes sub-Dicom Tags for more granular input parameters associated with each of the four example input parameter category listed. The last column provides an actual sub-tag ID for each sub-Dicom Tag. In various embodiments, the sub-Dicom tags (and/or their corresponding Tag IDs) correspond to the required input parameters for processing by the dose estimation model within each parameter input category. Table 300 also includes information describing the reason for each input parameter category, the parent Dicom tag or tags (where applicable) and their corresponding Tag IDs.

With reference to FIGs. 1-3B, in various embodiments, the new exam information 206 can provide only a subset of the input parameters associated with the respective input parameter categories illustrated in FIGs. 3A and 3B and additional input parameter categories noted above. In this regard, the dose management system identifies and extracts any required parameters for the dose estimation model 112 excluded from the new exam information 206 from the one or more target exams providing the closest match to the new exam information 206.

Continuing with process 200 at 216, the target dose estimation component 118 executes the dose estimation model 112 (i.e., the trained version of the dose estimation model 112). In other words, the target dose estimation component 118 applies the input parameters 214 as input to the dose estimation model 112 which in turn generates target dose information 218 as output. The target dose information 218 can vary depending on the modality of the exam and the body part imaged. As noted above, the target dose estimation 218 can include a target effective dose or target effective dose range, one or more target organ doses or dose ranges and/or specific dose measures or ranges tailored to respective imaging modalities, such as but not limited to, target DLP or target DLP range, target CTDI or target CTDI range, target SSDE or target SSDE range, target AGD or target AGD range, target DAP or target DAP range, target Kₐᵢᵣr or target Kₐᵢᵣr range, and the like. In various embodiments, at 220, the recommendation component 120 then sends (e.g., via communication component 106) the target dose information 218 to the medical imaging system 136_{N} scheduled for the exam and/or the RIS 138 prior to performance of the new medical imaging exam on the patient.

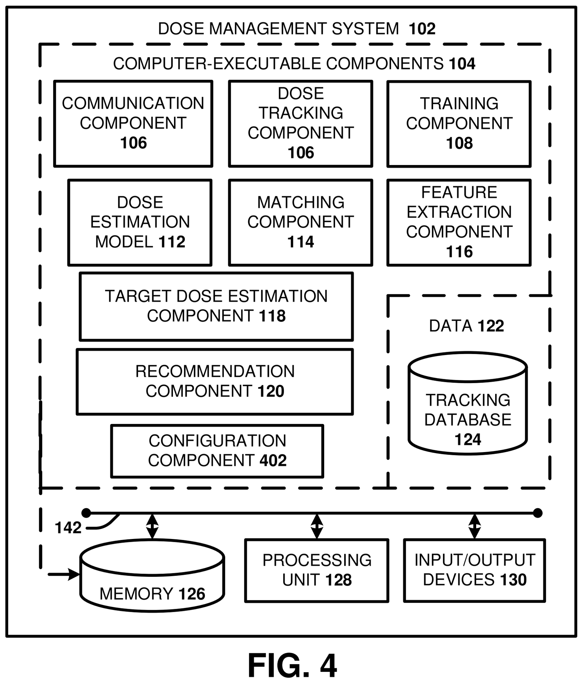

In various embodiments, based on provision of the target dose information 218 to the medical imaging system 136_{N}, the target dose information 218 can control performance of the new medical imaging exam in a manner that facilitates ensuring the actual amount of radiation exposed to the patient complies with the target dose information 218, as described with reference to FIG. 4.

In this regard, FIG. 4 illustrates another example embodiment of dose management system 102. In accordance with this embodiment, the computer-executable components 104 can further include configuration component 402. With reference to FIG. 4, in view of FIG. 3, in some embodiments, configuration component 402 can control the acquisition protocol and/or acquisition parameters used for the performance of the new medical imaging exam such that the actual radiation dose exposed to the patient complies with the target dose information 218.

In this regard, in some embodiments, the new exam information 206 includes an initial, recommended acquisition protocol for the exam and this initial acquisition protocol is used by the dose estimation model 112 to estimate the target dose information 218. In some implementations of these embodiments, based on reception of the target dose information 218 and the new exam information 206 including the initial acquisition protocol, configuration component 402 can interface with the medical imaging system 136_{N} and control programming (e.g., via a computer system coupled to the medical imaging system 136_{N}) of the acquisition protocol and/or acquisition parameters in accordance with the initial acquisition protocol. In other words, configuration component 402 can restrict manual adjustment (e.g., by the operating technician of the medical imaging system) of the acquisition protocol and parameters such that they comply with the initial acquisition protocol and/or parameters.

In some embodiments, the new exam information 206 may exclude the acquisition parameters and/or include only some of the acquisition parameters for the new medical imaging exam. In some implementations of these embodiments, the totality of the acquisition protocols and/or parameters involved in the type of the exam can be represented in the predefined input parameter processed by the dose estimation model 112. With these implementations, the excluded acquisition parameters from the new exam information 206 are extracted by the feature extraction component 116 from the one or more target exam reports for the one or more similar or matching historical exams and included in the input parameters 214 used by the dose estimation model 112 to estimate the target dose information 218. Additionally, or alternatively, the feature extraction component 116 may adjust (e.g., select and replace) one or more of the acquisition parameters to be used for the exam as included in the new exam information. With these embodiments, in addition to the target dose information 218, the recommendation component 120 can provide the totality of the acquisition protocol/parameters processed by the dose estimation model 112 to the medical imaging system 136_{N} prior to performance of the exam. In some implementations of these embodiments, based on reception of the target dose information 218 and the totality of the acquisition protocol/parameters evaluated by the dose estimation model 112, configuration component 402 can interface with the medical imaging system 136_{N} and control programming (e.g., via a computer system coupled to the medical imaging system 136_{N}) of the acquisition protocol and/or acquisition parameters in accordance with the totality of the acquisition protocol/parameters evaluated by the dose estimation model 112. In other words, the target dose management system 102 can restrict manual adjustment (e.g., by the operating technician of the medical imaging system) of the acquisition protocol and parameters such that they comply with the totality of the acquisition protocol and/or parameters evaluated by the dose estimation model.

In other embodiments, the medical imaging system 136 _{N} can include or correspond to an intelligent medical imaging system that monitors radiation exposure to patients during performance of the medical imaging exam in real-time and provides alerts in real-time at the medical imaging system 136 _{N} when dose levels exceed predefined thresholds. In accordance with these embodiments, based on provision of the target dose information 218 to the medical imaging system 136 _{N,} the medical imaging system 136 _{N} can be configured to apply the target dose information as the predefined thresholds. In other words, the target dose information 218 can control real-time alerts generated by the medical imaging system 136 _{N} regarding monitored radiation doses exposed to the patient during the exam in real-time.

Still in other embodiments, the medical imaging system 136 _{N} can include or correspond to an intelligent medical imaging system that includes software configured to estimate one or more radiation dose measures included in the target dose information 218 based on the acquisition parameters selected by the operating technician via the computer system coupled to the medical imaging system 136 _{N} at the time initiating set-up of the exam. Currently, these systems use generic, standardized, radiation dose thresholds to generate alerts when the estimated radiation doses exceed the standardized radiation dose thresholds. In accordance with these embodiments, the configuration component 402 can interface with the medical imaging system and set the thresholds used for such alerts in accordance with the target dose information 218 as opposed to the generic, standardized thresholds. In this regard, the target dose information 218 provides a significantly more accurate and patient specific tailored estimation of the optimal radiation dose ranges for the particular patient and the exam, as developed based on machine learning from a plethora of historical knowledge from actual past exams represented in the tracking database 122.

In some implementations of these embodiments, the configuration component 402 can also determine and suggest changes to the acquisition protocol and/or parameters that facilitate achieving the target dose information 218. For example, for a CT scan of the abdomen, the configuration component 402 may suggest no IV contrast for a particular patient.

In this regard, the dose management system 102 addresses controlling performance of the new imaging exams from the perspective of target radiation dose measures (e.g., optimal dose or ranges) determined for the respective exams under defined constraints for the respective exams. These defined constraints can be based on the order information and include or correspond to required or fixed parameters of each exam.

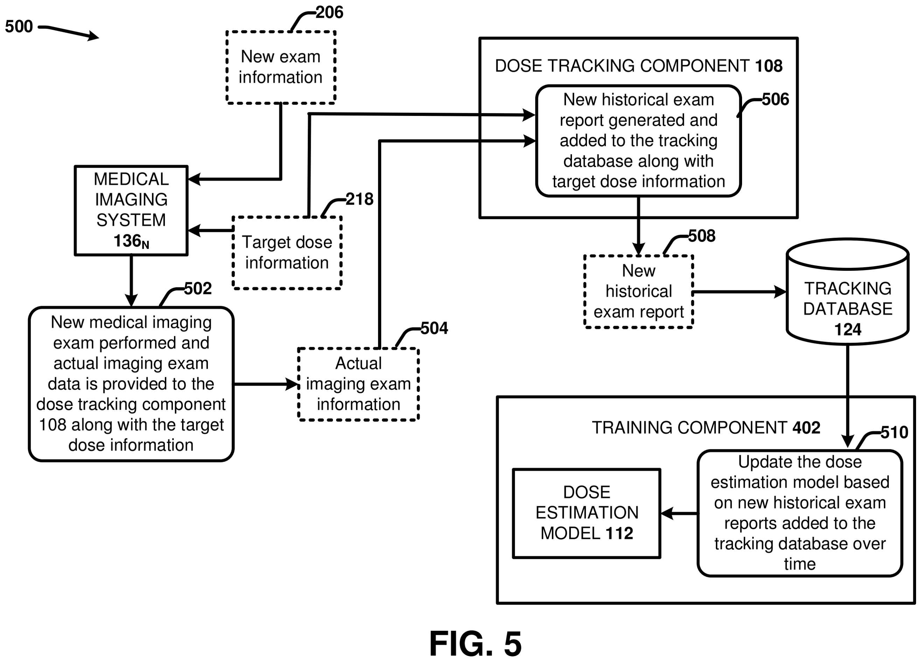

In addition, the target dose information 218 determined for respective exams can be used to optimize the performance of the dose estimation model over time based on difference between the actual radiation doses exposed to the patient determined after completion of the exams and the target dose information 218, as described below with reference to FIG. 5.

FIG. 5 illustrates an example process 500 that facilitates improving the performance of a dose estimation model over time, in accordance with one or more embodiments described herein. Process 500 continues the workflow described in FIG. 2. Process 500 beings at 502, wherein the new medical imaging exam is performed on the patient and actual imaging exam data 504 is provided to the dose tracking component 108 along with the target dose information 218. The actual imaging exam information can include or correspond to the detailed information that is included in the historical exam reports provided in the tracking database. In this regard, the actual exam information 504 can include one or more actual dose measures that reflect the actual radiation dose exposed to the patient during the new medical imaging exam as well as detailed information about the patient and the exam (e.g., exam type, modality, patient information, actual acquisition parameters used, image data, image quality information, smart observation information and so on). The actual imaging exam information 504 may be collected, extracted or otherwise received by the dose tracking component 108 from the medical imaging system 136_{N} itself, PACS 132, RIS 138 and/or EMR system 134. At 506, the dose tracking component generates a new historical exam report 508 for the exam and adds it to the tracking database 124 along with the target dose information 218. In this regard, the new historical exam report 508 can include or otherwise be associated with the target dose information 218 generated for the new exam and included in the tracking database.

At 510, the training component 402 updates the dose estimation model based on new historical exam report added to the tracking database 124 over time. For example, many new medical imaging exams can be performed at the respective medical imaging system 136_{1-N} daily, monthly, etc., with target dose information 218 determined for the respective exams via dose management system 102 using an initial trained version of the dose estimation model 112. Thus, over time, the tracking database 124 will include more and more new historical exam reports corresponding to new historical exam report 508 for a wide range of different exams with different patient profiles, modalities, acquisition parameter configurations and so on. In this regard, the training component 402 can update the dose estimation model 112 regularly or continuously over time based on differences between the target dose information and the actual dose information. For example, in some embodiments, the training component 402 can identify trends in the new historical exam reports with actual dose information that is less than the target dose information yet with acceptable quality measures. With these embodiments, the training component 402 can retrain the dose estimation model using these new historical exam reports to adjust the estimated target dose information for such exams in accordance with the lower dose trends, resulting in a new or updated version of the dose estimation model with improved performance relative to the previous version of the dose estimation model. In another example, the training component 402 can identify new historical exam reports with quality measures that are lower than an acceptable threshold yet comply within the target dose ranges estimated for the exams. With these embodiments, the training component 402 can retrain the dose estimation model to adjust or increase the lower threshold of the target dose ranges for such exams to provide an improved target does range estimation that results in improved quality. In addition, the training component can employ new historical exam reports that provide different input parameter configurations with respect to the original training data, such as different combinations of patient specific features, exam types, ROIs, clinical indications and the like, using select new historical exam reports with actual radiation dose measures that comply that do not comply with the target radiation dose measures to improve the estimations generated by the dose estimation model 112.

In addition to retraining and updating the dose estimation model 112, the new historical exams added to the tracking database 124 over time can further be used by the matching component 114 and the feature extraction component 116 in accordance with execution of a trained and/or updated version of the dose estimation model 112, as described with reference to FIG. 2.

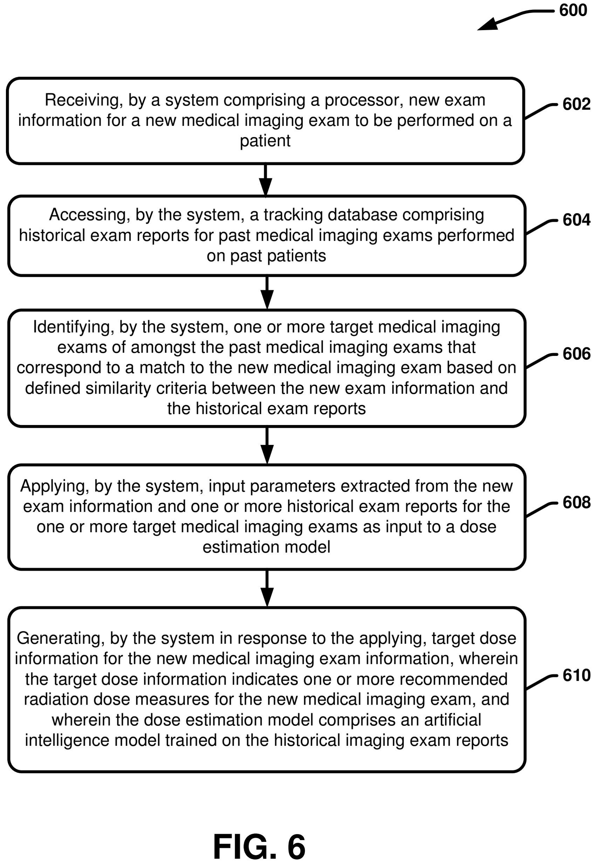

FIG. 6 illustrates an example computer-implemented method 600 that facilitates target radiation dose estimation for medical imaging exams leveraging AI, in accordance with one or more embodiments described herein. Method 600 comprises, at 602, receiving, by a system comprising a processor (e.g., dose management system 102), new exam information for a new medical imaging exam to be performed on a patient. At 604, method 600 comprises accessing, by the system (e.g., via matching component 114), a tracking database (e.g., tracking database 124) comprising historical exam reports for past medical imaging exams performed on past patients. At 606, method 600 comprises identifying, by the system (e.g., via matching component 114), one or more target medical imaging exams of amongst the past medical imaging exams that correspond to a match to the new medical imaging exam based on defined similarity criteria between the new exam information and the historical exam reports. At 608, method 600 comprises applying, by the system (e.g., via target dose estimation component 118), input parameters extracted from the new exam information and one or more historical exam reports for the one or more target medical imaging exams as input to a dose estimation model (e.g., dose estimation model 112). At 610, method 600 comprises generating, by the system in response to the applying, target dose information for the new medical imaging exam information, wherein the target dose information indicates one or more recommended radiation dose measures for the new medical imaging exam, and wherein the dose estimation model comprises an artificial intelligence model trained on the historical imaging exam reports. In various embodiments, the new exam information comprises a first subset of the input parameters, wherein the defined similarity criteria are based on the first subset, and wherein the input parameters further comprise a second subset of parameters extracted from the one or more historical exam reports. In some embodiments, the second subset comprises one or more acquisition parameters for the new exam.

In some embodiments, method 600 can further comprise, providing, by the system, the target dose information to an imaging system scheduled for the new imaging exam prior to performance of the new medical imaging exam on the patient (e.g., via recommendation component 120 and communication component 106). Method 600 can also further comprise controlling, by the system, usage of one or more acquisition parameters by the imaging system for the performance of the new medical imaging exam on the patient that facilitates achieving the one or more recommended radiation dose measures (e.g., via configuration component 402). For example, in addition to providing the target dose information to the imaging system, the configuration component 402 can also interface with the imaging system and provide the acquisition parameters selected by the feature extraction component 116 for usage for the exam. These are the acquisition parameters processed by the dose estimation model 112 in association with generating the one or more recommended radiation dose measures. The configuration component 402 can further automatically configure/enter the acquisition parameters into the computer operating system of the imaging system that controls acquisition as opposed to having the operating technician manually enter the acquisition parameters. The configuration component 402 can also restrict manual adjustment of the automatically entered acquisition parameters such that they comply with the recommended radiation dose measures.

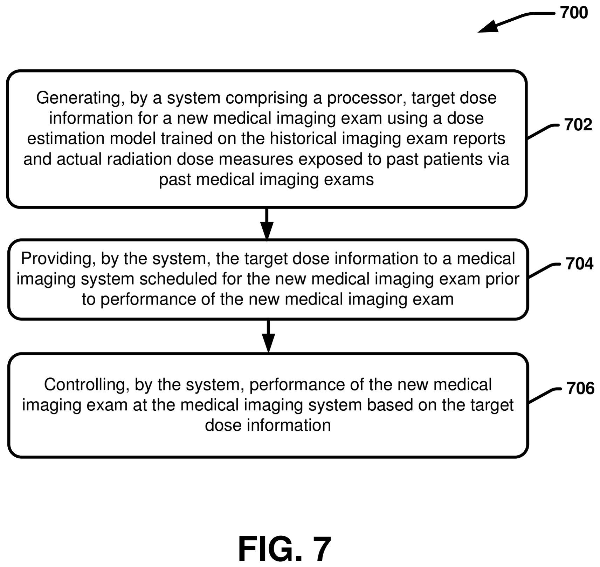

FIG. 7 illustrates another example computer-implemented method 700 that facilitates target radiation dose estimation for medical imaging exams leveraging AI, in accordance with one or more embodiments described herein. Method 700 comprises, at 702, generating, by a system comprising a processor (e.g., dose estimation system 102), target dose information for a new medical imaging exam using a dose estimation model (e.g., dose estimation model 112) trained on the historical imaging exam reports and actual radiation dose measures exposed to past patients via past medical imaging exams. At 704, method 700 comprises providing, by the system (e.g., via recommendation component 120 and communication component 106), the target dose information to a medical imaging system scheduled for the new medical imaging exam prior to performance of the new medical imaging exam. At 706, method 700 comprises, controlling by the system, performance of the new medical imaging exam at the medical imaging system based on the target dose information.

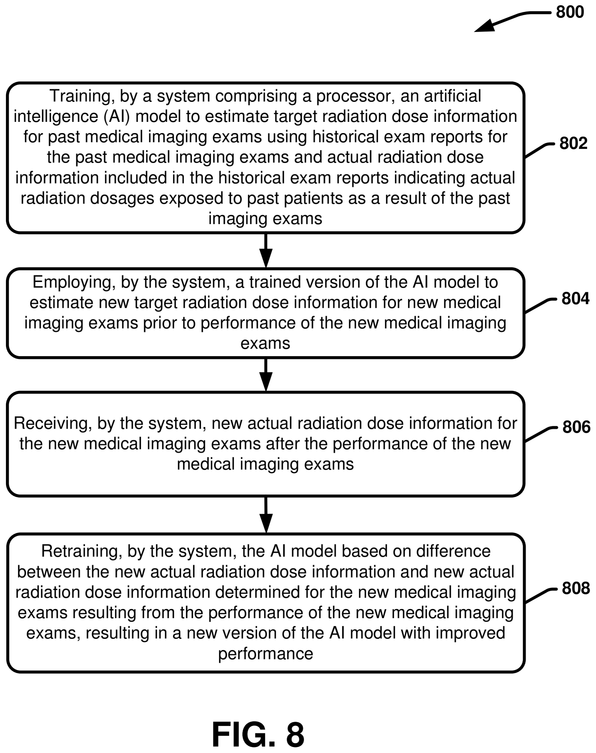

FIG. 8 illustrates another example computer-implemented method 800 that facilitates target radiation dose estimation for medical imaging exams leveraging AI, in accordance with one or more embodiments described herein. Method 800 comprises, at 802, training, by a system comprising a processor, an artificial intelligence (AI) model (e.g., dose estimation model 112) to estimate target radiation dose information for past medical imaging exams using historical exam reports for the past medical imaging exams and actual radiation dose information included in the historical exam reports indicating actual radiation dosages exposed to past patients as a result of the past imaging exams (e.g., via training component 108). At 804, method 800 comprises employing, by the system (e.g., via dose estimation component 118), a trained version of the AI model to estimate new target radiation dose information for new medical imaging exams prior to performance of the new medical imaging exams. At 806, method 800 comprises receiving, by the system, new actual radiation dose information for the new medical imaging exams after the performance of the new medical imaging exams. At 808, method 800 comprises retraining, by the system (e.g., via training component 108), the AI model based on difference between the new actual radiation dose information and new actual radiation dose information determined for the new medical imaging exams resulting from the performance of the new medical imaging exams, resulting in a new version of the AI model with improved performance.

In order to provide a context for the various aspects of the disclosed subject matter, FIGS. 9 and 10 as well as the following discussion are intended to provide a brief, general description of a suitable environment in which the various aspects of the disclosed subject matter may be implemented.

With reference to FIG. 9, a suitable environment 900 for implementing various aspects of this disclosure includes a computer 912. The computer 912 includes a processing unit 914, a system memory 916, and a system bus 918. The system bus 918 couples system components including, but not limited to, the system memory 916 to the processing unit 914. The processing unit 914 can be any of various available processors. Dual microprocessors and other multiprocessor architectures also can be employed as the processing unit 914.

The system bus 918 can be any of several types of bus structure(s) including the memory bus or memory controller, a peripheral bus or external bus, and/or a local bus using any variety of available bus architectures including, but not limited to, Industrial Standard Architecture (ISA), Micro-Channel Architecture (MSA), Extended ISA (EISA), Intelligent Drive Electronics (IDE), VESA Local Bus (VLB), Peripheral Component Interconnect (PCI), Card Bus, Universal Serial Bus (USB), Advanced Graphics Port (AGP), Personal Computer Memory Card International Association bus (PCMCIA), Firewire (IEEE 1394), and Small Computer Systems Interface (SCSI).

The system memory 916 includes volatile memory 920 and nonvolatile memory 922. The basic input/output system (BIOS), containing the basic routines to transfer information between elements within the computer 912, such as during start-up, is stored in nonvolatile memory 922. By way of illustration, and not limitation, nonvolatile memory 922 can include read only memory (ROM), programmable ROM (PROM), electrically programmable ROM (EPROM), electrically erasable programmable ROM (EEPROM), flash memory, or nonvolatile random access memory (RAM) (e.g., ferroelectric RAM (FeRAM). Volatile memory 920 includes random access memory (RAM), which acts as external cache memory. By way of illustration and not limitation, RAM is available in many forms such as static RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM.

Computer 912 also includes removable/non-removable, volatile/nonvolatile computer storage media. FIG. 9 illustrates, for example, a disk storage 924. Disk storage 924 includes, but is not limited to, devices like a magnetic disk drive, floppy disk drive, tape drive, Jaz drive, Zip drive, LS-90 drive, flash memory card, or memory stick. The disk storage 924 also can include storage media separately or in combination with other storage media including, but not limited to, an optical disk drive such as a compact disk ROM device (CD-ROM), CD recordable drive (CD-R Drive), CD rewritable drive (CD-RW Drive) or a digital versatile disk ROM drive (DVD-ROM). To facilitate connection of the disk storage devices 924 to the system bus 918, a removable or non-removable interface is typically used, such as interface 926.

FIG. 9 also depicts software that acts as an intermediary between users and the basic computer resources described in the suitable operating environment 900. Such software includes, for example, an operating system 928. Operating system 928, which can be stored on disk storage 924, acts to control and allocate resources of the computer system 912. System applications 930 take advantage of the management of resources by operating system 928 through program modules 932 and program data 934, e.g., stored either in system memory 916 or on disk storage 924. It is to be appreciated that this disclosure can be implemented with various operating systems or combinations of operating systems.

A user enters commands or information into the computer 912 through input device(s) 936. Input devices 936 include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processing unit 914 through the system bus 918 via interface port(s) 938. Interface port(s) 938 include, for example, a serial port, a parallel port, a game port, and a universal serial bus (USB). Output device(s) 940 use some of the same type of ports as input device(s) 936. Thus, for example, a USB port may be used to provide input to computer 912, and to output information from computer 912 to an output device 940. Output adapter 942 is provided to illustrate that there are some output devices 940 like monitors, speakers, and printers, among other output devices 940, which require special adapters. The output adapters 942 include, by way of illustration and not limitation, video and sound cards that provide a means of connection between the output device 940 and the system bus 918. It should be noted that other devices and/or systems of devices provide both input and output capabilities such as remote computer(s) 944.

Computer 912 can operate in a networked environment using logical connections to one or more remote computers, such as remote computer(s) 944. The remote computer(s) 944 can be a personal computer, a server, a router, a network PC, a workstation, a microprocessor based appliance, a peer device or other common network node and the like, and typically includes many or all of the elements described relative to computer 912. For purposes of brevity, only a memory storage device 946 is illustrated with remote computer(s) 944. Remote computer(s) 944 is logically connected to computer 912 through a network interface 948 and then physically connected via communication connection 950. Network interface 948 encompasses wire and/or wireless communication networks such as local-area networks (LAN), wide-area networks (WAN), cellular networks, etc. LAN technologies include Fiber Distributed Data Interface (FDDI), Copper Distributed Data Interface (CDDI), Ethernet, Token Ring and the like. WAN technologies include, but are not limited to, point-to-point links, circuit switching networks like Integrated Services Digital Networks (ISDN) and variations thereon, packet switching networks, and Digital Subscriber Lines (DSL).

Communication connection(s) 950 refers to the hardware/software employed to connect the network interface 948 to the bus 918. While communication connection 950 is shown for illustrative clarity inside computer 912, it can also be external to computer 912. The hardware/software necessary for connection to the network interface 948 includes, for exemplary purposes only, internal and external technologies such as, modems including regular telephone grade modems, cable modems and DSL modems, ISDN adapters, and Ethernet cards.

FIG. 10 is a schematic block diagram of a sample-computing environment 1000 with which the subject matter of this disclosure can interact. The system 1000 includes one or more client(s) 1010. The client(s) 1010 can be hardware and/or software (e.g., threads, processes, computing devices). The system 1000 also includes one or more server(s) 1030. Thus, system 1000 can correspond to a two-tier client server model or a multi-tier model (e.g., client, middle tier server, data server), amongst other models. The server(s) 1030 can also be hardware and/or software (e.g., threads, processes, computing devices). The servers 1030 can house threads to perform transformations by employing this disclosure, for example. One possible communication between a client 1010 and a server 1030 may be in the form of a data packet transmitted between two or more computer processes.

The system 1000 includes a communication framework 1050 that can be employed to facilitate communications between the client(s) 1010 and the server(s) 1030. The client(s) 1010 are operatively connected to one or more client data store(s) 1020 that can be employed to store information local to the client(s) 1010. Similarly, the server(s) 1030 are operatively connected to one or more server data store(s) 1040 that can be employed to store information local to the servers 1030.

It is to be noted that aspects or features of this disclosure can be exploited in substantially any wireless telecommunication or radio technology, e.g., Wi-Fi; Bluetooth; Worldwide Interoperability for Microwave Access (WiMAX); Enhanced General Packet Radio Service (Enhanced GPRS); Third Generation Partnership Project (3GPP) Long Term Evolution (LTE); Third Generation Partnership Project 2 (3GPP2) Ultra Mobile Broadband (UMB); 3GPP Universal Mobile Telecommunication System (UMTS); High Speed Packet Access (HSPA); High Speed Downlink Packet Access (HSDPA); High Speed Uplink Packet Access (HSUPA); GSM (Global System for Mobile Communications) EDGE (Enhanced Data Rates for GSM Evolution) Radio Access Network (GERAN); UMTS Terrestrial Radio Access Network (UTRAN); LTE Advanced (LTE-A); etc. Additionally, some or all of the aspects described herein can be exploited in legacy telecommunication technologies, e.g., GSM. In addition, mobile as well non-mobile networks (e.g., the Internet, data service network such as internet protocol television (IPTV), etc.) can exploit aspects or features described herein.

While the subject matter has been described above in the general context of computer-executable instructions of a computer program that runs on a computer and/or computers, those skilled in the art will recognize that this disclosure also can or may be implemented in combination with other program modules. Generally, program modules include routines, programs, components, data structures, etc. that perform particular tasks and/or implement particular abstract data types. Moreover, those skilled in the art will appreciate that the inventive methods may be practiced with other computer system configurations, including single-processor or multiprocessor computer systems, mini-computing devices, mainframe computers, as well as personal computers, hand-held computing devices (e.g., PDA, phone), microprocessor-based or programmable consumer or industrial electronics, and the like. The illustrated aspects may also be practiced in distributed computing environments where tasks are performed by remote processing devices that are linked through a communications network. However, some, if not all aspects of this disclosure can be practiced on stand-alone computers. In a distributed computing environment, program modules may be located in both local and remote memory storage devices.

As used in this application, the terms "component," "system," "platform," "interface," and the like, can refer to and/or can include a computer-related entity or an entity related to an operational machine with one or more specific functionalities. The entities disclosed herein can be either hardware, a combination of hardware and software, software, or software in execution. For example, a component may be, but is not limited to being, a process running on a processor, a processor, an object, an executable, a thread of execution, a program, and/or a computer. By way of illustration, both an application running on a server and the server can be a component. One or more components may reside within a process and/or thread of execution and a component may be localized on one computer and/or distributed between two or more computers.

In another example, respective components can execute from various computer readable media having various data structures stored thereon. The components may communicate via local and/or remote processes such as in accordance with a signal having one or more data packets (e.g., data from one component interacting with another component in a local system, distributed system, and/or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form.

As used herein, the terms "example" and/or "exemplary" are utilized to mean serving as an example, instance, or illustration. For the avoidance of doubt, the subject matter disclosed herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

Various aspects or features described herein can be implemented as a method, apparatus, system, or article of manufacture using standard programming or engineering techniques. In addition, various aspects or features disclosed in this disclosure can be realized through program modules that implement at least one or more of the methods disclosed herein, the program modules being stored in a memory and executed by at least a processor. Other combinations of hardware and software or hardware and firmware can enable or implement aspects described herein, including a disclosed method(s). The term "article of manufacture" as used herein can encompass a computer program accessible from any computer-readable device, carrier, or storage media. For example, computer readable storage media can include but are not limited to magnetic storage devices (e.g., hard disk, floppy disk, magnetic strips...), optical discs (e.g., compact disc (CD), digital versatile disc (DVD), blu-ray disc (BD) ...), smart cards, and flash memory devices (e.g., card, stick, key drive...), or the like.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor may also be implemented as a combination of computing processing units.

In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory and/or memory components described herein can be either volatile memory or nonvolatile memory, or can include both volatile and nonvolatile memory.

By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or methods herein are intended to include, without being limited to including, these and any other suitable types of memory.

It is to be appreciated and understood that components, as described with regard to a particular system or method, can include the same or similar functionality as respective components (e.g., respectively named components or similarly named components) as described with regard to other systems or methods disclosed herein.

What has been described above includes examples of systems and methods that provide advantages of this disclosure. It is, of course, not possible to describe every conceivable combination of components or methods for purposes of describing this disclosure, but one of ordinary skill in the art may recognize that many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

## Claims

1. A system, comprising:
at least one memory that stores computer-executable components; and
at least one processor that executes the computer-executable components stored in the at least one memory, wherein the computer-executable components comprise:
a communication component that receives new exam information for a new medical imaging exam to be performed on a patient;
a matching component that accesses a tracking database comprising historical exam reports for past medical imaging exams performed on past patients and identifies one or more target medical imaging exams of amongst the past medical imaging exams that correspond to a match to the new medical imaging exam based on defined similarity criteria between the new exam information and the historical exam reports;
a target dose estimation component that applies input parameters extracted from the new exam information and one or more historical exam reports for the one or more target medical imaging exams as input to a dose estimation model, and generates target dose information for the new medical imaging exam as output, wherein the target dose information indicates one or more recommended radiation dose measures for the new medical imaging exam;
a recommendation component that provides the target dose information to an imaging system scheduled for the new imaging exam prior to performance of the new medical imaging exam on the patient; and
a configuration component that controls usage of one or more acquisition parameters by the imaging system for the performance of the new medical imaging exam on the patient that facilitates achieving the one or more recommended radiation dose measures.

2. The system of claim 1, wherein the dose estimation model comprises an artificial intelligence model trained on the historical imaging exam reports.

3. The system of claim 1, wherein the input parameters comprise the one or more acquisition parameters.

4. The system of claim 1, wherein the input parameters are selected from the group consisting of: modality, local study description, anatomical region, series description, series type, the one or more acquisition parameters, patient factors, actual console configuration, presence and concentration, image quality, repeat element and dose justification.

5. The system of claim 1, wherein the new exam information comprises a first subset of the input parameters, wherein the defined similarity criteria are based on the first subset, and wherein the input parameters further comprise a second subset of parameters extracted from the one or more historical exam reports.

6. The system of claim 5, wherein the first subset is selected from the group consisting of: modality, local series description, anatomical region, and patient factors, and wherein the patient factors comprise patient size, patient age and patient gender, and wherein the second subset comprises the one or more acquisition parameters.

7. The system of claim 1, wherein the one or more recommended radiation dose measures comprise effective dose and organ dose.

8. The system of claim 1, wherein the computer-executable components further comprise:
a feature extraction component that extracts the input parameters from the new exam information and the one or more historical exam reports.

9. The system of claim 1, wherein the computer-executable components further comprise:
a training component that trains the dose estimation model using the historical exam reports and actual radiation dose information included in the historical exam reports indicating actual radiation dosages exposed to the past patients as a result of the past imaging exams.

10. The system of claim 9, wherein the computer-executable components further comprise:
a dose tracking component that associates new target dose information generated by the system for new medical imaging exams with new historical exam reports generated for the new medical imaging exams and stored in the tracking database following performance of the new medical imaging exams, and wherein the training component tunes and updates the dose estimation model based on differences between the new target dose information and new actual radiation dose information included in the new historical exam reports, resulting in an improved version of the dose estimation model.

11. A method, comprising:
receiving, by a system comprising a processor, new exam information for a new medical imaging exam to be performed on a patient;
accessing, by the system, a tracking database comprising historical exam reports for past medical imaging exams performed on past patients;
identifying, by the system, one or more target medical imaging exams of amongst the past medical imaging exams that correspond to a match to the new medical imaging exam based on defined similarity criteria between the new exam information and the historical exam reports;
applying, by the system, input parameters extracted from the new exam information and one or more historical exam reports for the one or more target medical imaging exams as input to a dose estimation model;
generating, by the system in response to the applying, target dose information for the new medical imaging exam information, wherein the target dose information indicates one or more recommended radiation dose measures for the new medical imaging exam;
providing, by the system, the target dose information to an imaging system scheduled for the new imaging exam prior to performance of the new medical imaging exam on the patient; and
controlling, by the system, usage of one or more acquisition parameters by the imaging system for the performance of the new medical imaging exam on the patient that facilitates achieving the one or more recommended radiation dose measures.

12. The method of claim 11, wherein the one or more recommended radiation dose measures comprise effective dose and organ dose.

13. The method of claim 11, further comprising:
training, by the system, the dose estimation model using the historical exam reports and actual radiation dose information included in the historical exam reports indicating actual radiation dosages exposed to the past patients as a result of the past imaging exams.

14. The method of claim 13, further comprising:
associating, by the system, new target dose information generated by the system for new medical imaging exams with new historical exam reports generated for the new medical imaging exams and stored in the tracking database following performance of the new medical imaging exams; and
updating, by the system, the dose estimation model based on differences between the new target dose information and new actual radiation dose information included in the new historical exam reports, resulting in an improved version of the dose estimation model.

15. A non-transitory machine-readable storage medium, comprising executable instructions that, when executed by a processor, facilitate performance of operations, comprising:
receiving new exam information for a new medical imaging exam to be performed on a patient;
accessing a tracking database comprising historical exam reports for past medical imaging exams performed on past patients;
identifying one or more target medical imaging exams of amongst the past medical imaging exams that correspond to a match to the new medical imaging exam based on defined similarity criteria between the new exam information and the historical exam reports;
applying input parameters extracted from the new exam information and one or more historical exam reports for the one or more target medical imaging exams as input to a dose estimation model; and
generating, in response to the applying, target dose information for the new medical imaging exam information, wherein the target dose information indicates one or more recommended radiation dose measures for the new medical imaging exam, and wherein the dose estimation model comprises an artificial intelligence model trained on the historical imaging exam reports.
